(19) Europäisches Patentamt / European Patent Office / Office européen des brevets

(11) **EP 4 201 322 A1**

(12) **DEMANDE DE BREVET EUROPEEN**

(43) Date de publication:
28.06.2023 Bulletin 2023/26

(21) Numéro de dépôt: **23156594.6**

(22) Date de dépôt: **20.10.2015**

(51) Classification Internationale des Brevets (IPC):
**A61B 5/107** (2006.01)    **A61C 19/04** (2006.01)
**G06T 17/00** (2006.01)

(52) Classification Coopérative des Brevets (CPC):
**A61C 19/045; A61B 5/1079; G06T 19/00;**
A61C 9/008; G06T 2210/41

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.10.2014 FR 1460071**

(62) Numéro(s) de document de la (des) demande(s)
initiale(s) en application de l'article 76 CBE:
**15791329.4 / 3 209 204**

(71) Demandeur: **Modjaw
69100 Villeurbanne (FR)**

(72) Inventeur: **JAISSON, Maxime
73800 Les Marches (FR)**

(74) Mandataire: **Regimbeau
20, rue de Chazelles
75847 Paris Cedex 17 (FR)**

Remarques:
Cette demande a été déposée le 14-02-2023 comme
demande divisionnaire de la demande mentionnée
sous le code INID 62.

(54) **PROCEDE ET SYSTEME DE LOCALISATION DES POINTS CONDYLIENS D'UN PATIENT**

(57) L'invention concerne un procédé de localisation des points condyliens (3) d'un patient, comprenant :
- l'obtention d'un modèle surfacique tridimensionnel du visage du patient,
- l'identification d'éléments caractéristiques du visage du patient sur ledit modèle surfacique tridimensionnel du visage du patient,
- à partir desdits éléments caractéristiques, la détermination, sur ladite image stéréoscopique, respectivement ledit modèle surfacique tridimensionnel du visage du patient, de points, axes et plans de référence du visage du patient,
- l'obtention d'un modèle tridimensionnel (Ms) de l'arcade dentaire maxillaire et d'un modèle tridimensionnel (Mi) de l'arcade dentaire mandibulaire du patient,
- le recalage des modèles tridimensionnels des arcades dentaires par rapport aux plans de référence (A, B, C) du modèle surfacique tridimensionnel du patient,
- l'enregistrement de la cinématique mandibulaire du patient,
- l'application de la cinématique mandibulaire enregistrée aux modèles tridimensionnels des arcades dentaires recalés, pour animer lesdits modèles tridimensionnels (Ms, Mi),
- à partir de ladite animation des modèles tridimensionnels (Ms, Mi) des arcades mandibulaires, la localisation du point condylien droit, respectivement gauche, ledit point condylien étant défini comme l'intersection d'un axe de rotation (R1) d'un mouvement d'ouverture-fermeture et d'un axe de rotation (R2) d'un déplacement latéral de la mandibule vers la droite, respectivement vers la gauche.

FIG. 6B

EP 4 201 322 A1

## Description

### DOMAINE DE L'INVENTION

La présente invention concerne un procédé de localisation des points condyliens d'un patient, ainsi qu'un système pour la mise en oeuvre dudit procédé.

### ARRIERE PLAN DE L'INVENTION

Lorsque l'organe dentaire est dégradé, le rôle du chirurgien-dentiste est de le restaurer.

Lors d'un délabrement important, cette restauration fait appel à des artifices prothétiques remplaçant tout ou partie d'une ou des dents concernées.

Cela ne peut se faire sans prendre en considération l'occlusion dentaire qui est la façon dont s'organisent les contacts interdentaires et inter-arcades.

Dans ce contexte, la gestion des contacts interdentaires est un impératif thérapeutique.

Il existe par ailleurs d'autres situations dans lesquelles la gestion des contacts dentaires est un impératif thérapeutique.

Par exemple, lorsque les dents sont en position ectopique, un traitement orthodontique est entrepris de sorte à déplacer les dents grâce à un appareil. Le schéma et la distribution des contacts occlusaux changent et des règles sont à respecter dans ce cas pour ne pas nuire au patient.

Dans d'autres cas, lorsque des troubles de l'articulation ou de contraction musculaire sont diagnostiqués et ont un rapport avec l'occlusion, le chirurgien-dentiste peut, par la conception d'une gouttière occlusale, atténuer voire corriger ces dysfonctionnements.

Par extension, l'occlusion assure l'interface entre les deux maxillaires. Leur confrontation est possible grâce à un os mobile : la mandibule (maxillaire inférieur).

La qualité de cette occlusion est primordiale et doit assurer 3 fonctions essentielles (centrage ; calage ; guidage) de cette même mandibule afin de préserver les structures environnantes (articulation, muscles...).

La motilité de la mandibule est due à une articulation, l'articulation temporo-mandibulaire (ATM) et une mise en mouvement par la contraction des muscles masticateurs.

A tout moment, le chirurgien-dentiste est soucieux de la préservation de la bonne santé de ces composants mais également de son rétablissement lorsque des pathologies sont objectivées (telles que myalgie, arthropathie). Dans ce cas, le chirurgien-dentiste peut, par le biais d'une réhabilitation de l'occlusion, avoir un effet rétroactif sur les pathologies de l'appareil manducateur.

La construction ou reconstruction de l'occlusion est sous l'influence de certains déterminants, qui sont des données liées au patient ayant une influence sur l'anatomie occlusale.

Ces déterminants sont importants à appréhender dans certaines situations car l'artisan prothésiste, en les maîtrisant, s'en inspire pour modeler la surface occlusale des dents.

Les déterminants de l'occlusion sont les facteurs qui influencent l'occlusion. Ces facteurs sont divisés en deux groupes: les déterminants fixes et les déterminants susceptibles d'être modifiés par le remodelage ou le repositionnement des dents.

Les déterminants modifiables sont :

- la forme des dents (hauteur des cuspides, profondeur des fosses...)
- la position des dents,
- la dimension verticale,
- les courbes occlusales.

Les déterminants fixes sont :

(1) le positionnement vertical et horizontal des arcades par rapport au déterminant postérieur,
(2) l'écartement condylien
(3) le positionnement antéropostérieur des arcades par rapport au déterminant postérieur articulaire,
(4) le déterminant postérieur articulaire, qui est défini par :

• la pente condylienne
• l'angle de Bennett
• le mouvement initial de Bennett.

Ces déterminants fixes et modifiables sont interdépendants les uns des autres.

Les déterminants modifiables sont ceux sur lesquelles le chirurgien-dentiste concentre son diagnostic et ses travaux de réhabilitation. Pour parfaire le diagnostic et ainsi assurer une réhabilitation optimum pour le patient, une étude des déterminants fixes et des interdépendances entre déterminants fixes et modifiables est nécessaire.

A cet effet, il existe sur le marché des outils appelés articulateurs qui simulent plus ou moins bien la physiologie de l'appareil manducateur.

Ces articulateurs sont disponibles sur le marché sous la forme mécanique ou numérique. Ces simulateurs reproduisent une cinématique mandibulaire permettant la prise en compte les déterminants fixes dans l'analyse diagnostique. Le paramétrage de ces simulateurs s'effectue en utilisant des approximations des déterminants fixes 2, 3 et 4. L'anatomie de l'articulation temporo-mandibulaire est simulée grâce à une addition de valeurs angulaires schématisant les trajets du condyle mandibulaire dans l'espace. Cela est paramétré mécaniquement au niveau des boîtiers condyliens du simulateur.

Cependant, du fait de leur conception et de leur mode de fonctionnement, les simulateurs sont source d'erreurs. De plus, ils ne permettent qu'une maîtrise approximative des déterminants, cela au prix d'une programmation fastidieuse et d'une manutention coûteuse au cabinet dentaire. En outre, la cinématique mandibulaire recréée à partir de ces simulateurs n'est qu'une reproduction approximative des réels mouvements mandibulaires.

Une conséquence directe est l'externalisation chez le prothésiste de cette tâche pourtant déterminante car garante d'un traitement prothétique fiable, confortable et durable.

Le document WO 2013/030511 décrit un procédé de conception d'un appareil dentaire qui met en oeuvre un enregistrement de la cinématique mandibulaire du patient.

Ce procédé comprend tout d'abord soit l'obtention d'une image volumique du massif facial par une technique de tomodensitométrie, soit la détermination des plans de référence du massif facial par repérage de points d'intérêt sur le visage du patient.

Par ailleurs, des modèles tridimensionnels des arcades dentaires du patient sont obtenus. Lesdits modèles, positionnés l'un par rapport à l'autre lors de leur création, sont recalés avec l'image volumique du massif facial ou les plans de référence déterminés au préalable.

L'enregistrement de la cinématique mandibulaire est mis en oeuvre en équipant le patient d'un marqueur fixé sur le front du patient et de marqueurs fixés directement sur les dents de l'arcade mandibulaire ou sur la mandibule par l'intermédiaire d'un support, et en repérant et enregistrant les déplacements desdits marqueurs au moyen d'une caméra lors de mouvements mandibulaires du patient.

Cependant l'obtention des éléments permettant de positionner les modèles des arcades dentaires l'un par rapport à l'autre reste à améliorer. En effet, la tomodensitométrie suppose d'exposer le patient à des rayons X et l'on cherche à minimiser l'exposition du patient à de tels rayons.

En ce qui concerne la solution alternative consistant à déterminer les plans de référence du massif facial, elle requiert un certain nombre de manipulations du praticien pour pointer les différents points d'intérêt.

**BREVE DESCRIPTION DE L'INVENTION**

Un but de l'invention est de proposer un procédé de localisation des points condyliens d'un patient.

Comme on le verra en détail plus bas, ce procédé comprend entre autre un véritable enregistrement de la cinématique mandibulaire qui, une fois modélisée grâce à un logiciel, permet d'animer les modèles numériques des arcades dentaires du patient. La prise en compte des déterminants se fait donc de façon simple et intuitive.

En outre, l'information fournie par les aspects logiciel et matériel de l'invention est beaucoup plus exhaustive et est une représentation réelle des données morphologiques et morpho-dynamiques du patient.

Le procédé offre l'avantage d'être fait en temps réel au cabinet du praticien, assurant ainsi une étude complète et sur mesure du patient.

Parmi les informations disponibles, on retrouve la position des arcades dentaires dans l'espace par rapport aux articulations et au massif facial. Son intérêt est d'étudier (en cas d'analyse occlusale) et de reconstruire les courbes occlusales. Ces courbes, la courbe de SPEE et la courbe de WILSON qualifient l'organisation intra arcade. De façon simplifiée, ces courbes correspondent à la façon dont s'orientent les surfaces occlusales des dents, leurs cuspides et les bords incisifs dans l'espace. Cela porte à notre connaissance les possibilités de répartition des forces et de rencontre interarcade. Il en découle une analyse sur la forme individuelle de chaque dent, son anatomie propre, la profondeur des sillons, la hauteur cuspidienne, sa position propre par rapport aux autres dents voisines et antagonistes.

Conformément à l'invention, il est proposé un procédé et un système selon les revendications annexées.

**BREVE DESCRIPTION DES DESSINS**

D'autres caractéristiques et avantages de l'invention ressortiront de la description détaillée qui va suivre, en référence aux dessins annexés sur lesquels :

- la figure 1 est un schéma de principe d'une acquisition stéréoscopique,
- la figure 2 est un schéma de principe en vue de dessus de l'ensemble capteur / lentille / objet lors d'une acquisition stéréoscopique,
- les figures 3A et 3B présentent une partie des points, axes et plans de référence, représentés sur un modèle 3D du visage du patient,
- la figure 4 illustre le placement de contours des éléments caractéristiques de la face (nez, bouche, oreilles, yeux) sur le modèle surfacique 3D du visage du patient,
- la figure 5 présente le principe de détermination du point condylien sur le modèle surfacique 3D du visage du patient,
- les figures 6A et 6B illustrent le principe du pointage permettant le recalage des arcades dentaires avec le modèle 3D du visage du patient,
- la figure 7 illustre le principe de la détection des dents par reconnaissance faciale,
- la figure 8 illustre le principe de l'enregistrement de la cinématique mandibulaire du patient,
- la figure 9 est une vue de l'écran d'affichage,
- les figures 10A et 10B illustrent le principe de localisation du point condylien droit par une étude cinématique.

**DESCRIPTION DETAILLEE DE L'INVENTION**

Le procédé de modélisation de la cinématique mandibulaire d'un patient comprend principalement les étapes suivantes :

- modélisation surfacique 3D du visage par acquisition au moyen d'une caméra stéréoscopique et reconstruction, la reconstruction pouvant être réalisée suivant différentes formes d'exécution, notamment :

    - la reconstruction stéréoscopique, dérivée de la photogrammétrie,
    - la reconstruction à partir de lumière structurée projetée sur le visage à l'aide d'un vidéoprojecteur,
    - la modélisation à partir d'un faisceau laser projeté sur le visage du patient,

- placement des points, axes et plans de référence du visage, mettant en oeuvre un procédé de reconnaissance faciale ;
- positionnement des arcades dentaires par rapport à ces plans, lesdits plans étant associés ou non au modèle surfacique 3D du visage (détermination des déterminants fixes 1 et 3) ;
- enregistrement et étude de la cinématique mandibulaire (détermination du déterminant 4) ;
- éventuellement, incorporation des structures osseuses.

Ce procédé est mis en oeuvre par ordinateur.

Le système permettant la mise en oeuvre du procédé comprend :

- une caméra stéréoscopique, éventuellement équipée d'émetteur infrarouges, d'un vidéoprojecteur ou d'un projecteur laser ;
- un ensemble de marqueurs détectables par la caméra stéréoscopique, comprenant un dispositif de fixation dudit ensemble à la tête du patient (par exemple, cet ensemble se présente sous la forme d'un casque destiné à être fixé sur le front du patient),
- un ordinateur couplé à la caméra, comprenant un processeur pour faire fonctionner des algorithmes de traitement des images et de modélisation mentionnés plus bas,
- une mémoire pour enregistrer les images acquises et les modèles utilisés,
- un écran d'affichage pour permettre au praticien de visualiser les différentes étapes mises en oeuvre au cours du procédé.

Modélisation 3D du visage du patient

*Cas 1 : par acquisition stéréoscopique*

Cette modélisation met en oeuvre une caméra stéréoscopique pour acquérir une image du visage du patient. Grâce au traitement informatique de l'image stéréoscopique, on obtient une reconstruction tridimensionnelle du visage du patient.

Par ailleurs, si la caméra stéréoscopique est en couleur, le modèle 3D peut être texturé automatiquement. Si la caméra est en noir et blanc, la texture peut être obtenue grâce à une photographie couleur importée dans un second temps au niveau du logiciel. Des points de corrélation sont choisis entre l'image et le modèle pour plaquer ensuite la photographie sur le modèle en distribuant les informations colorimétriques.

Afin de retranscrire la vision humaine, une caméra stéréoscopique utilise deux capteurs et deux objectifs pour simuler les yeux. Le logiciel de reconstruction cherche ensuite dans les deux images l'objet regardé pour calculer les angles de convergence. La caméra stéréoscopique utilisée est une caméra pré-calibrée. Le processus de rectification consiste à replacer chaque pixel de l'image à sa place théorique permettant ainsi de compenser les défauts d'alignement des capteurs, ainsi que les différents effets et distorsions des optiques. La nouvelle position de chaque pixel est obtenue par calibration de la caméra lors de la mise au point de celle-ci. La calibration consiste à présenter aux deux caméras des images comportant des lignes horizontales et verticales parfaitement identifiables. Le logiciel cherche les lignes et les intersections de ligne dans chaque paire d'image. Il déplace ensuite chaque pixel de chaque image pour que les intersections de points soient les plus alignées possibles et correspondent ligne à ligne entre la caméra gauche et la caméra droite. Ce calcul est automatisé par le logiciel de calibration.

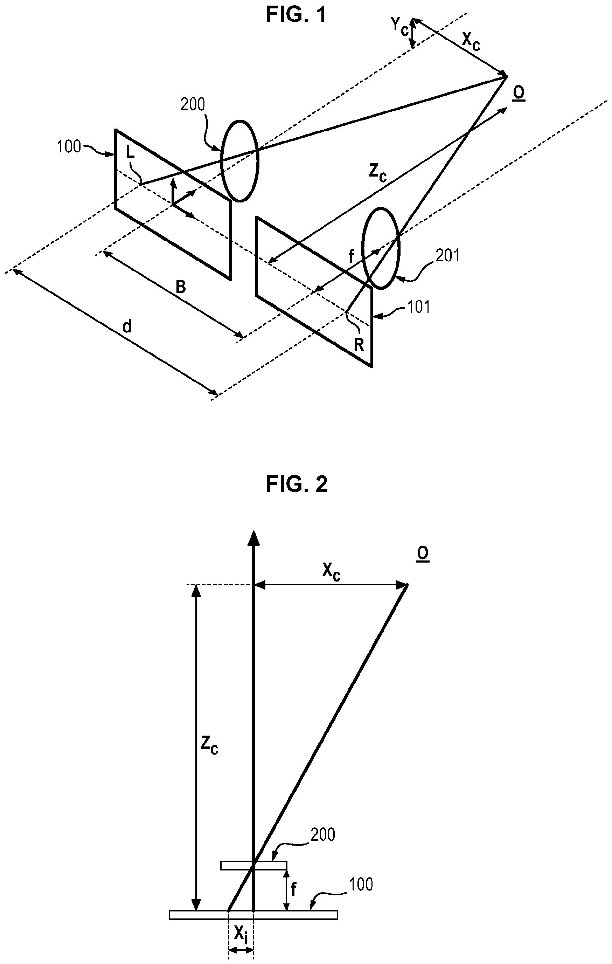

La figure 1 illustre deux capteurs 100, 101 supposés parfaits, parfaitement alignés, dans un plan regardant un objet O à travers des objectifs 200, 201 parfaits.

La distance d qui sépare l'objet O sur les deux images correspond à l'angle de convergence humain. On peut d'ailleurs calculer la distance Zc entre les capteurs 100, 101 et l'objet O par le calcul suivant :

$$Zc = \frac{B * f}{(L - R)} = \frac{B * f}{d}$$

avec :

- B : la distance inter orbitale,
- f : la distance focale, et
- L et R la position respective de l'objet dans chaque image.

On nomme (Xi, Yi) la position de l'objet dans l'image de gauche, et (Xc, Yc, Zc) la position de l'objet dans le plan caméra gauche.

La figure 2 est un schéma en vue de dessus de l'ensemble capteur 100 / objectif 200 / objet O.

On peut écrire :

$$\frac{Xi}{Xc} = \frac{f}{Zc}$$

On peut donc calculer Xc en fonction de Xi, Zc et f :

$$Xc = \frac{Xi * Zc}{f}$$

De la même façon, Yc s'exprime ainsi :

$$Yc = \frac{Yi * Zc}{f}$$

La position (Xc, Yc, Zc) de l'objet O est donc entièrement connue.

Pour calculer une carte de profondeur, il faut ensuite identifier quel pixel de l'image de droite correspond à quel pixel de l'image de gauche.

Pour cela, il existe plusieurs méthodes.

La méthode la plus simple est de chercher pour chaque image de gauche quel pixel de l'image de droite a la même valeur. Afin de limiter l'erreur, l'algorithme prend en compte non seulement le pixel en lui-même mais aussi les huit pixels autour. Cette méthode est appelé la somme des absolues différences (SAD). Cette méthode est classiquement utilisée pour retrouver un motif dans une image. Elle a pour principal inconvénient sa grande sensibilité aux variations de luminosité. Si par exemple l'image de gauche est plus lumineuse que l'image de droite, il sera difficile de trouver des pixels avec exactement la même valeur. Cette solution est pourtant très répandue car simple à mettre en place. Elle est plutôt conseillée pour les éclairages intérieurs maîtrisés.

Une alternative est de transformer l'image de façon à ne garder que les variations de lumière d'un pixel à l'autre et non plus la valeur réelle. L'algorithme classiquement utilisé s'appelle la transformée de Census. Il permet d'obtenir deux images identiques même si elles n'ont pas été prises avec exactement les mêmes conditions lumineuses. Il faut ensuite chercher pour chaque pixel de l'image de gauche quel pixel de l'image de droite a la même valeur. Cet algorithme s'appelle le calcul de la distance de Hamming.

Une fois que l'on a identifié quel pixel de l'image de gauche correspond à quel pixel de l'image de droite, on peut créer la carte de profondeur. On crée donc une image dont la valeur de chaque pixel correspond à la distance en nombre de pixels entre le pixel de gauche et son correspondant dans l'image de droite.

Dans ce cas et pour la présente invention, il est possible de réaliser des acquisitions stéréoscopiques du visage et d'en récupérer un maillage de surface. Un algorithme de recalage automatique est utilisé pour associer les maillages entre eux dans la mesure où les acquisitions ont été faites selon différents angles de vue (face, profil, ¾...).

Ce maillage forme le modèle 3D du visage du patient qui est utilisé dans la suite du procédé.

Pour faciliter le recalage, la caméra stéréoscopique peut être équipée de composants électroniques supplémentaires comme des gyroscopes accéléromètres capteurs inertiels. Dans le cas où la caméra est déplacée autour du visage du patient, une information de déplacement est fournie au logiciel pour déterminer l'angle de vue par rapport à l'image initiale et ainsi faciliter l'association des surfaces entre elles.

Cas 2 : *par projection de lumière structurée*

A cet effet, on projette sur le visage du sujet une série de motifs lumineux (traits, carrés, ronds,...) au moyen d'un vidéoprojecteur, créant des images déformées de ces motifs sur le visage du patient. En fonction de la distance jusqu'à la surface, les motifs prennent des formes différentes en raison du positionnement décalé du projecteur et de la caméra stéréoscopique. Ensuite un logiciel vient en déduire la forme du visage en fonction de l'ensemble des surfaces déformées dont il dispose et de la distance entre les points composant ce motif.

*Cas 3 : par projection et balayage laser*

A cet effet, on projette avec un balayage sur le visage du sujet plusieurs bandes laser qui sont également vues par les objectifs de la caméra stéréoscopique pour situer chaque point. En fonction de la distance jusqu'à une surface, le point apparaît à un endroit différent dans le champ de vision des différents objectifs. Cette technique est appelée triangulation parce que chaque point des bandes laser, l'objectif et l'émetteur laser forment un triangle. La longueur d'un côté dudit triangle, à savoir la distance entre l'objectif et l'émetteur laser est connue. L'angle du côté de l'émetteur laser est également connu. L'angle du côté de l'objectif peut être déterminé en regardant l'emplacement du point laser dans le champ de vision de la caméra. Ces trois données déterminent la forme et les dimensions du triangle et donnent la position de chaque point des bandes laser.

Détermination des plans de référence du visage par reconnaissance faciale

Afin de placer les points, axes et plans de référence du visage, on fait appel à des algorithmes de détection automatique appliqués au modèle 3D déterminé précédemment ou à une image (noir et blanc ou couleur) du visage du patient.

Les points de référence ont pour intérêt de caractériser la morphologie du visage du patient afin de guider le diagnostic et de donner des références architecturales pour la modélisation et la mise en place du traitement.

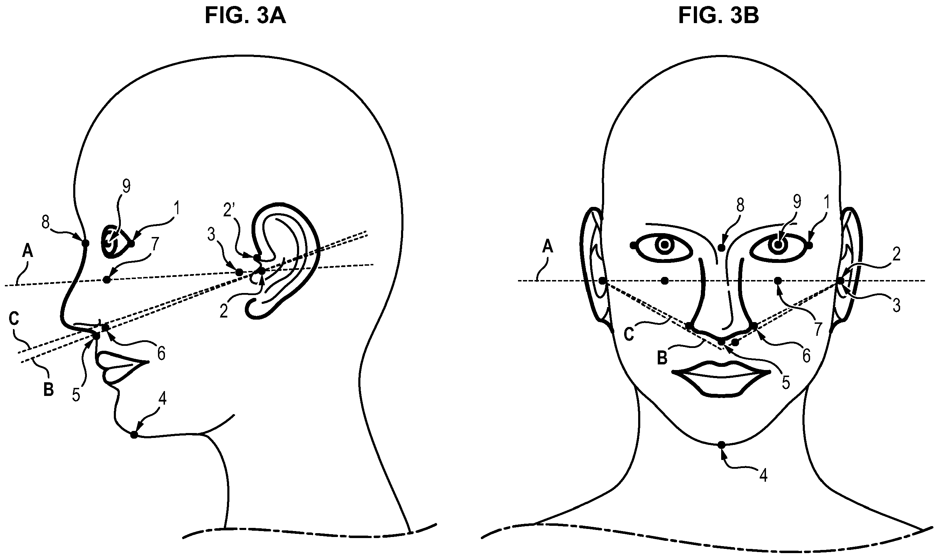

En référence aux figures 3A et 3B, les points de référence sont les suivants :

1. Ectocanthion : angle latéral de l'œil, où se rejoignent les paupières.

2. Tragion : Point le plus haut du tragus de l'oreille dans le plan sagittal

2'. Sommet de l'angle du tragus.

3. Point condylien ou d'émergence de l'axe charnière (un droit, un gauche) ; comme indiqué plus bas, plusieurs définitions de ce point sont possibles

4. Menton : point le plus inférieur du menton cutané.

5. Point sous nasal : à la base du nez, point le plus haut et le plus reculé de l'encoche naso-labiale

6. Aile du nez : point de jonction entre l'aile du nez et la lèvre supérieure (un droit, un gauche)

7. Point infra-orbitaire : point le plus déclive du rebord inférieur de l'orbite (un droit, un gauche)

8. Nasion cutané : point de l'ensellure nasale ; dans le plan sagittal, c'est, sur le profil cutané, le point le plus reculé de l'encoche fronto-nasale.

9. Point pupillaire : Point situé au centre de la pupille (un droit, un gauche).

Cette liste n'est pas exhaustive et peut être enrichie par l'utilisateur.

L'ensemble de ces points peuvent être placés sur la représentation du visage du patient de différentes manières qui sont décrites plus bas.

Ces points sont réunis pour générer les plans et les axes de référence, à savoir :

A. Plan axio-orbitaire : Plan passant par le point condylien et le plan infra-orbitaire

B. Plan de camper : Plan passant par le tragion et le point sous-nasal

C. Plan aile du nez tragion.

D'autres plans et axes de référence, non référencés sur les figures 3A et 3B sont :

- le plan sagittal médian passant par le nasion, le point sous nasal et le point situé à équidistance des points condyliens
- l'axe bicondylien : axe passant par le point condylien gauche et le point condylien droit
- l'axe bi-pupillaire : axe passant par les points pupillaires droits et gauche.

L'ensemble de ces points est placé soit sur le modèle surfacique 3D du patient si celui-ci a été construit au préalable, soit sur une image stéréoscopique du visage du patient en noir et blanc ou en couleur, la construction du modèle 3D du visage du patient étant réalisée ensuite. Trois modes de réalisation sont exposés ci-après.

### Cas 1 : à partir de l'acquisition 3D du visage du patient

Ce mode de réalisation utilise une modélisation 3D surfacique du visage du patient obtenue grâce au procédé de reconstruction stéréoscopique décrit plus haut.

Sur le modèle surfacique 3D obtenu, on peut plaquer et déformer un modèle rassemblant les contours des différents éléments caractéristiques de la face pour englober les yeux, la bouche les oreilles et le nez.

La déformation se fait quelle que soit l'orientation du modèle dans l'espace (face, profil, etc.).

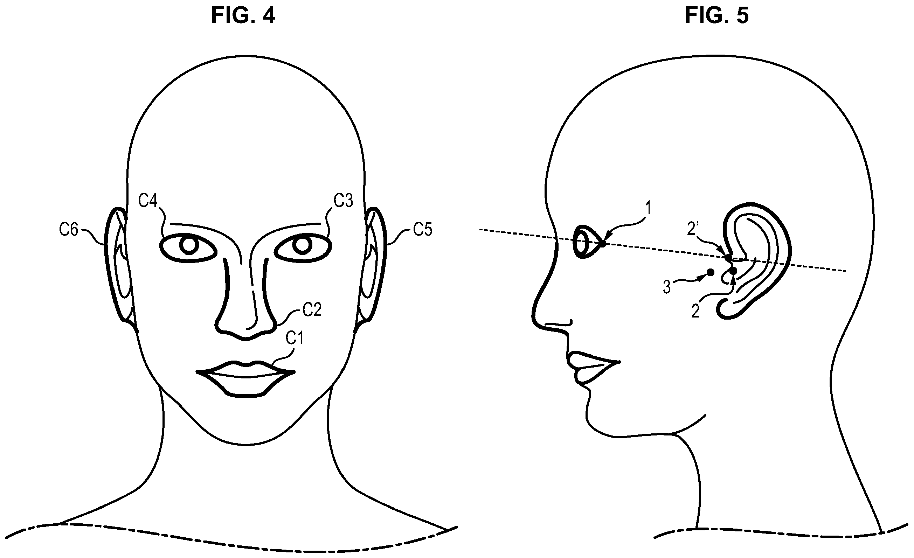

Ces contours C1, C2, C3, C4, C5, C6 ne sont pas forcément affichés comme sur la figure 4, mais ils sont en tout état de cause associés au maillage pour faciliter la détection des points de référence.

Le positionnement des points, axes et plans de référence est alors appliqué par le logiciel au modèle surfacique 3D, en utilisant l'emplacement des éléments caractéristiques du visage.

### Cas 2 : à partir d'images en noir et blanc

La caméra stéréoscopique est composée de deux objectifs pouvant fournir des images en noir et blanc.

Premièrement il y a la prise de vue photo ou vidéo du visage.

Puis le logiciel analyse différents aspects de cette image pour mettre en évidence les caractéristiques individuelles propres au visage pour lequel il est programmé (les yeux, la position du nez, la forme du menton, l'oreille...). Les points de référence décrits plus haut sont placés ensuite à l'aide de ces repères. Le procédé stéréoscopique détermine ensuite la position de chaque point dans l'espace.

Ce procédé fait appel à plusieurs techniques dont celle dite Eigenface. Dans un premier temps, l'image du visage est décomposée par le logiciel en plusieurs images utilisant des nuances de gris. Chacune de ces images fait alors ressortir une caractéristique particulière. Il est associé à cette technique la technique de l'analyse des caractéristiques ajoutant à chacune des images la position et la distance entre les points s'appuyant sur l'analyse des variations de luminosité des éléments anatomiques du visage (nez, oeil, oreille, bouche). Cette analyse se fait par exemple de face, de profil et de 3/4. Un modèle déformable est appliqué aux différentes images.

### Cas 3 : à partir d'images en couleur

Le même procédé que dans le cas 2 peut être appliqué aux images en couleur. Le logiciel décompose les images couleur en nuances de gris. C'est le cas lorsque des caméras couleur sont utilisées.

L'utilisation de la distribution des pixels couleurs RGB (« red green blue ») permet d'affiner l'application du modèle déformable sur l'image et cela dans les trois plans de l'espace permis par la stéréoscopie.

Une fois les plans, axes, et points de références trouvés et validés, leur position est ensuite figée dans le repère du casque fixé sur la tête du patient.

*Détermination du point condylien*

Le point condylien 3 ne peut pas être obtenu directement car il ne correspond pas à un élément caractéristique visible sur une image ou un modèle de la face.

La situation dans l'espace de ce point peut se faire sur le modèle 3D du visage obtenu par reconstruction 3D ou sur les images 2D issues des objectifs de la caméra stéréoscopique, à partir des autres points de référence déterminés au préalable.

Comme illustré sur la figure 5, le positionnement du point condylien 3 dépend de la situation de deux autres points : l'ectocanthion 1 et le sommet 2' de l'angle du tragus. Par convention, le point condylien 3 est situé 10 mm en avant et 5 mm en bas de la ligne reliant le sommet de l'angle du tragus à l'ectocanthion.

Un autre procédé de détermination du point condylien, mettant en oeuvre la cinématique mandibulaire, sera exposé plus bas.

*Association de la réalité augmentée*

De manière optionnelle, une fois le calcul réalisé par le logiciel pour placer les points et les éléments caractéristiques, ceux-ci peuvent être affichés en temps réel à l'écran grâce à la vidéo de la caméra stéréoscopique.

Il s'affiche à l'écran le visage du patient surmonté des plans et axes de référence sans avoir besoin de placer sur le patient le casque frontal.

Détermination du positionnement des arcades dentaires par rapport au massif facial et à l'axe bicondylien

Cette technique remplace l'utilisation de l'outil mécanique appelé arc facial (facebow).

L'objectif est de reproduire de manière numérique le véritable positionnement des arcades du patient par rapport au massif osseux.

Le procédé consiste donc à utiliser les différentes technologies susmentionnées afin de placer les modèles maxillaire et mandibulaire numérisés par rapport aux plans et points de référence caractérisant le massif facial. Orientation et position des modèles des arcades dentaires sont ainsi obtenues.

Deux modes de réalisation permettant le positionnement des arcades dentaires sont décrits ci-dessous.

*Cas 1 : pointage des points sur l'arcade dentaire du patient*

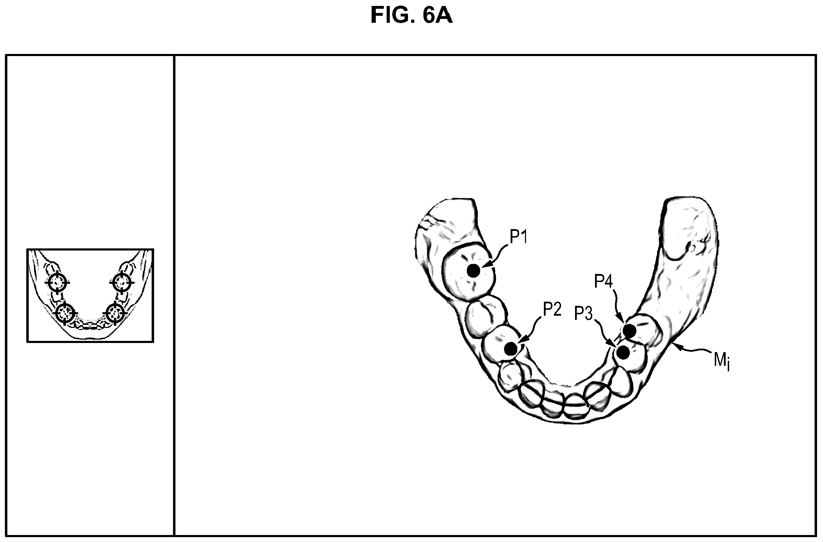

Les points à rechercher sont placés de manière aléatoire sur le modèle 3D des arcades dentaires, que l'arcade soit partiellement dentée, qu'elle présente des dents préparées ou des piliers implantaires ou que l'arcade soit édentée (cf. figure 6A, où l'on voit quatre points P1, P2, P3 et P4 sur le modèle Mi de l'arcade mandibulaire).

On utilise un pointeur muni de marqueurs détectables par la caméra stéréoscopique.

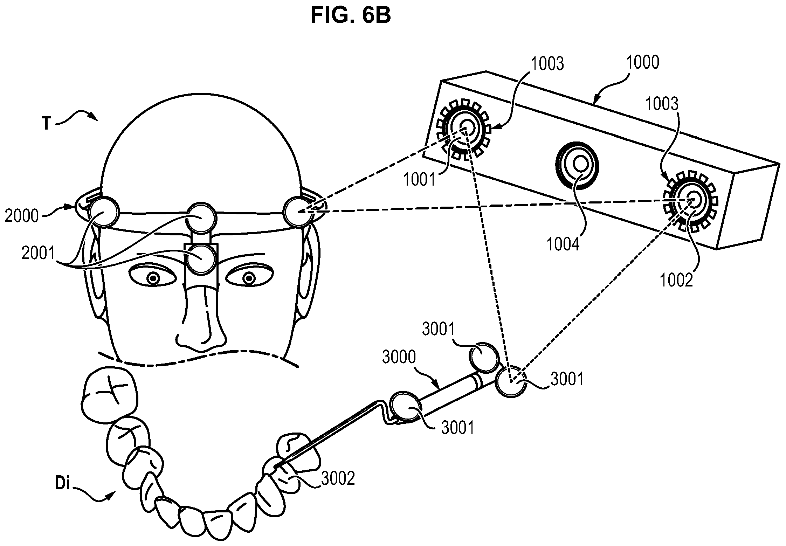

Dans cette étape, le praticien veille à placer précisément l'embout du pointeur dans la bouche du patient sur l'arcade mandibulaire Di aux mêmes endroits que sur le modèle virtuel (cf. figure 6B).

La figure 6B illustre un mode de réalisation des moyens matériels du système de modélisation.

Ledit système comprend une caméra stéréoscopique 1000 présentant deux objectifs 1001, 1002. De manière optionnelle, la caméra 1000 comprend en outre des émetteurs de lumière infrarouge 1003 agencés par exemple autour des objectifs 1000, 1001. De manière optionnelle, la caméra 1000 comprend un vidéoprojecteur 1004 permettant de projeter de la lumière structurée ou un émetteur laser permettant de projeter des bandes laser sur le visage du patient en vue de la modélisation décrite plus haut.

Le système comprend en outre un casque frontal 2000 destiné à être placé sur la tête T du patient. Ce casque frontal supporte une pluralité de marqueurs 2001 visibles par la caméra stéréoscopique 1000. Lorsque la caméra est pourvue des émetteurs infrarouge 1003, les marqueurs 2001 sont avantageusement réalisés en un matériau réfléchissant, la lumière infrarouge servant alors à augmenter la visibilité des marqueurs 2001 par la caméra 1000. Naturellement, la forme du casque et le nombre de marqueurs ne sont illustrés sur la figure 6B qu'à titre indicatif et l'homme du métier pourra choisir un autre support et un autre agencement des marqueurs sans sortir du cadre de la présente invention. Par ailleurs, toute technologie de marqueur visible par la caméra peut être employée, notamment des diodes, des mires noires et blanches ou en couleur ou des sphères, des pastilles ou d'autres objets réfléchissants.

Pour le mode de réalisation consistant à positionner les arcades dentaires sur le modèle 3D du visage du patient par pointage, le système comprend en outre un pointeur 3000 portant des marqueurs 3001 visibles par la caméra stéréoscopique 1000. Lorsque la caméra est pourvue des émetteurs infrarouge 1003, les marqueurs 3001 sont avantageusement réalisés en un matériau réfléchissant, la lumière infrarouge servant alors à augmenter la visibilité des marqueurs 3001 par la caméra 1000. Naturellement, la forme du pointeur et le nombre de marqueurs ne sont illustrés sur la figure 6B qu'à titre indicatif et l'homme du métier pourra choisir une autre forme de pointeur et un autre agencement des marqueurs sans sortir du cadre de la présente invention. Par ailleurs, toute technologie de marqueur visible par la caméra peut être employée, notamment des diodes, des mires noires et blanches ou en couleur ou des sphères, des pastilles ou d'autres objets réfléchissants.

En utilisation, les marqueurs 3001 du pointeur 3000 sont repérés par la caméra 1000.

Dès que le praticien a positionné l'embout 3002 du pointeur sur un point visé, il effectue une action visant à enregistrer la position de l'extrémité 3002 du pointeur dans l'espace. Cette action peut être par exemple une pression du pied sur une pédale de commande, une pression du doigt sur un commutateur, ou encore un masquage des marqueurs par la main du praticien (liste non limitative). A chacune de ces actions, la position des points ciblés est vue par la caméra stéréoscopique. Lorsque les points à pointer sélectionnés sur le modèle 3D ont été situés sur l'arcade dentaire Di et validés grâce au pointeur, le modèle 3D prend sa place par rapport au référentiel.

Au préalable les arcades dentaires ont été scannées dans une relation d'occlusion d'engrènement connu et reproductible. L'outil utilisé est une caméra d'empreinte optique intrabuccale. On scanne une arcade puis l'autre, ainsi qu'une empreinte vestibulaire (sur le côté) des dents en engrènement pour connaître la position d'une arcade par rapport à l'autre. Cette opération est connue en elle-même et ne fait pas en tant que telle partie de l'invention. Une autre méthode consisterait à utiliser un scanner de table. Celui-ci scanne les modèles en plâtre issus d'empreintes physicochimiques l'un après l'autre puis en position d'engrènement. L'invention peut en effet être mise en oeuvre avec tout modèle 3D des arcades dentaires généré par les techniques disponibles sur le marché.

Grâce à l'opération préalable ayant permis de connaître la situation des plans et axe de référence, la position du maxillaire par rapport au plan de référence est donc ensuite déduite. De la même façon, le modèle 3D du maxillaire suit les mouvements de la tête par application de la réalité augmentée ou grâce à l'utilisation du casque frontal mentionné plus haut.

*Cas 2 : détection des dents par reconnaissance faciale*

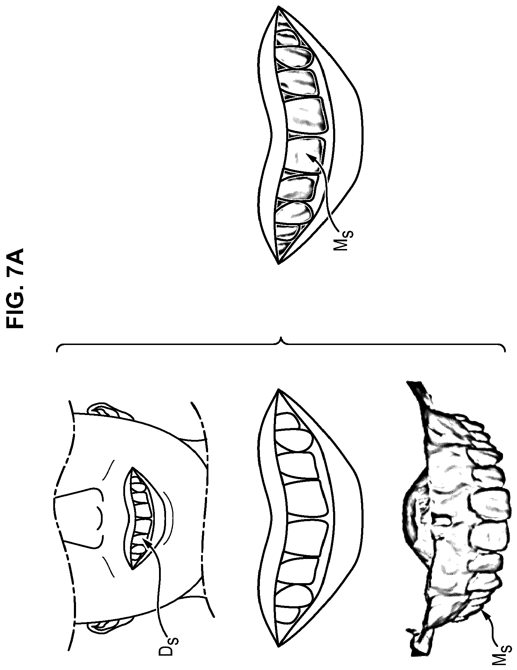

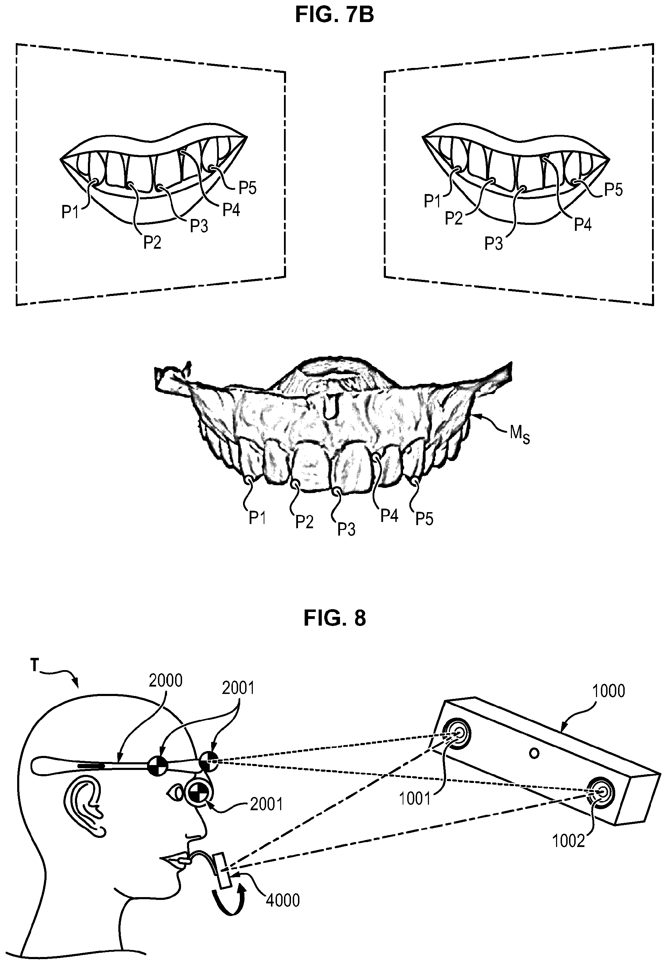

Comme illustré sur les figures 7A et 7B, la caméra stéréoscopique peut visualiser, si l'accès aux dents est autorisé par des écarteurs, ou lors d'un sourire forcé, le positionnement de l'arcade dentaire supérieure (vue du haut à gauche de la figure 7A, montrant les dents de l'arcade maxillaire Ds). Une fois la carte de profondeur obtenue pour les dents maxillaires visibles ou lorsque les lignes caractéristiques des dents ont été relevée par reconnaissance faciale (vue du milieu à gauche de la figure 7A), il est possible d'associer sur le modèle 3D du visage ou sur l'image vidéo, le modèle 3D de cette même arcade obtenu par scanner optique (vue du bas à gauche de la figure 7A, montrant le modèle 3D Ms de l'arcade maxillaire)..

Le recalage se fait soit automatiquement à partir des points caractéristiques des dents grâce à une analyse géométrique par relevé de points saillants, coins, contours, lignes de transition, etc. (vue de droite de la figure 7A).

Le modèle 3D est ensuite intégré et déformé grâce à une matrice de transformation pour se recaler sur l'image du visage ou sur sa modélisation.

Il peut se faire manuellement en appliquant sur l'image ou le modèle 3D du visage ainsi que sur le modèle 3D de l'arcade dentaire des points de recalage P1 à P5 (cf. figure 7B).

Une fois la position du modèle 3D trouvée et validée, elle est ensuite figée par la technique de la réalité augmentée ou par l'application du casque frontal placé sur la tête du patient. Tout comme le sont les plans, axes, et points de références.

Enregistrement et étude de la cinématique mandibulaire

Comme illustré sur la figure 8, on met en place sur la mandibule du patient une arche 4000 fixée aux dents portant des marqueurs détectables par une caméra.

Le patient est par ailleurs également équipé du casque frontal 2000 mentionné plus haut, et portant des marqueurs 2001 détectables par ladite caméra.

De préférence, on utilise dans cette étape la caméra stéréoscopique 1000 utilisée dans les précédentes étapes du procédé. Ainsi, un seul matériel d'acquisition d'images est suffisant pour mettre en oeuvre l'ensemble du procédé, ce qui simplifie l'équipement nécessaire et en limite le coût.

Il reste cependant possible d'utiliser tout autre type de caméra du marché, sous réserve d'équiper l'arche fixée sur la mandibule des marqueurs détectables par ladite caméra.

*Enregistrement de la cinématique*

La position statique du modèle mandibulaire par rapport au maxillaire (RIM, relation intermaxillaire) a été enregistrée au préalable en bouche par une caméra optique intrabuccale ou au niveau d'un scanner de table au laboratoire. Il faut désormais connaître la façon dont se déplace la mandibule dans l'espace. Le principe est le même que précédemment. Des marqueurs sont placés uniquement sur les dents mandibulaires.

Ici le traqueur est composé de diodes suivies par la caméra. Mais cela peut être des mires noires et blanches ou couleurs ou des sphères, des pastilles ou d'autres objets réfléchissants. Le déplacement des marqueurs de la mandibule est suivi par la caméra 1000 et cela par rapport aux marqueurs 2001 du front. Une transformation rigide permet de déduire le mouvement du modèle de l'arcade mandibulaire 3D par rapport au modèle 3D maxillaire.

La caméra assure le suivi des marqueurs placés sur le référentiel frontal 2000 et celui des marqueurs solidaires de l'arcade mandibulaire en mouvement. La fixation des marqueurs se fait par le biais de l'arche 4000 ou d'une gouttière.

Le modèle de l'arcade maxillaire et les plans de référence sont associés à l'animation de la mandibule en mouvement. Dans le logiciel il est possible d'afficher ou de cacher chacun de ces éléments.

L'étude des contacts en dynamique est réalisée avec les mêmes outils que pour l'étude en statique. Dans ce cas on obtient une information sur la répartition des conflits inter-arcades dans le temps et dans l'espace.

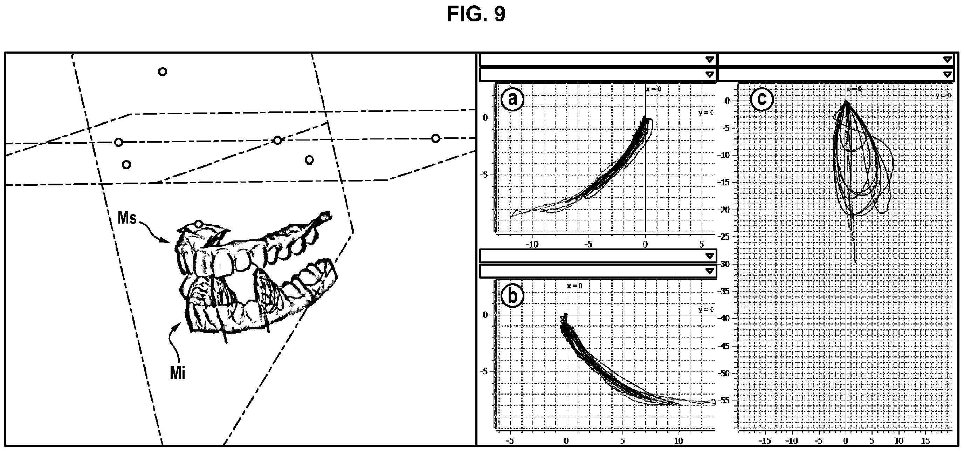

La figure 9 est une vue de l'écran d'affichage présentant des exemples de visualisations obtenues. La partie de gauche présente le modèle Ms de l'arcade maxillaire et le modèle Mi de l'arcade mandibulaire par rapport aux plans de référence du visage du patient. Dans la partie de droite, la zone a affiche le tracé du mouvement du condyle gauche dans le plan sagittal, la zone b affiche le tracé du mouvement du condyle droit dans le plan sagittal et la zone c affiche le tracé du mouvement du dentalé dans le plan frontal.

*Localisation du point condylien par étude cinématique*

Idéalement, le point condylien doit correspondre à un point de la tête condylienne qui reste immobile lors des mouvements de rotation pure de la mandibule.

Cette rotation pure est retrouvée lors de l'ouverture buccale dans les 15 à 20 premiers millimètres et dans les mouvements de latéralité au niveau des condyles pivotants, c'est-à-dire se trouvant du côté du déplacement de la mandibule.

Un point étant au minimum une intersection de deux droites, il faut donc déterminer deux axes de rotation correspondant à l'ouverture buccale et aux mouvements de latéralité droite et gauche.

On part de la loi physique décrivant le déplacement d'un corps solide dans l'espace : le déplacement d'un corps rigide entre deux points peut être décrit comme une rotation autour d'un axe et d'une translation le long de cet axe.

Dans le procédé mis en oeuvre dans la présente invention, on cherche d'abord à placer l'axe de rotation correspondant au mouvement d'ouverture-fermeture.

Cet axe autour duquel le mouvement mandibulaire se produit peut être déterminé mathématiquement. A cet effet, trois points sont d'abord choisis sur le modèle de l'arcade dentaire inférieure et créent un système de coordonnées de référence. L'axe de rotation est trouvé en résolvant automatiquement par un algorithme la matrice de transformation représentant le déplacement de ces trois points entre la position bouche fermée et la bouche ouverte entre 15 et 20 mm. Pour situer l'axe de rotation et résoudre la matrice de transformation, on fait l'hypothèse que la mandibule effectue un mouvement de rotation pure. Sinon, il y a un nombre infini d'axes de rotation chacun avec un vecteur de translation différent.

Le calcul est le même en faisant l'acquisition du mouvement de latéralité et en résolvant la matrice de transformation représentant le déplacement de trois points placés sur le modèle mandibulaire entre la position bouche fermée et la position de latéralité extrême.

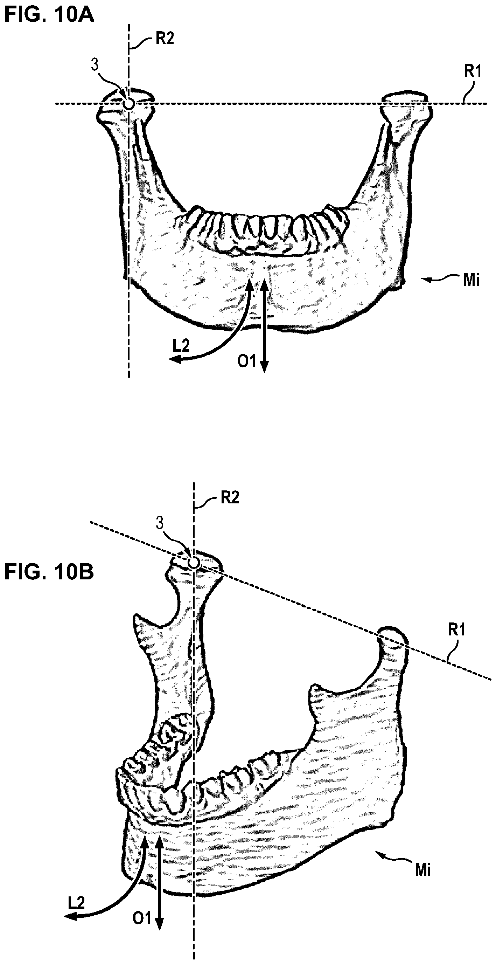

Les figures 10A et 10B illustrent ce principe de localisation du point condylien, respectivement sur une vue de face et une vue de côté de la mandibule. On s'intéresse sur ces figures au point condylien droit 3, qui se trouve à l'intersection des deux axes de rotation :

- l'axe de rotation R1 qui est déterminé un mouvement d'ouverture fermeture de 20 mm d'amplitude 20mm (double flèche O1),
- l'axe de rotation R2 qui est obtenu par un mouvement de latéralité droite (double flèche L2).

*Protocole une fois le patient équipé du système de captation de mouvement*

- Demander au patient d'ouvrir et fermer la bouche avec un degré d'amplitude de 15-20mm de façon répétée. Pour exécuter ce mouvement, le patient peut être guidé par le dentiste qui vient saisir le menton et guider ces mouvements.
- Demander au patient de décaler le menton le plus loin possible sur le côté droit et de répéter ce mouvement.
- Demander au patient de décaler le menton le plus loin possible sur le côté gauche et de répéter ce mouvement.

Le logiciel affiche le point condylien droit qui correspond à l'intersection entre l'axe de rotation droit en latéralité droite avec l'axe de rotation de l'ouverture fermeture.

Le logiciel affiche le point condylien gauche qui correspond à l'intersection entre l'axe de rotation gauche en latéralité gauche avec l'axe de rotation de l'ouverture fermeture.

*Incorporation des structures osseuses*

Le modèle 3D des structures osseuses n'est pas nécessaire dans le procédé pour placer les arcades dentaires dans l'espace et par rapport au massif facial.

Cependant, l'intégration de ce modèle peut être intéressante pour quelques applications, notamment :

1) pour affiner l'examen diagnostic lors de l'étude de la cinématique

2) pour rendre l'étude de la cinématique plus conviviale et intuitive

3) pour obtenir l'information de volume osseux et du rapport des racines des dents avec celui-ci avant le traitement orthodontique et planifier ce même traitement

4) pour obtenir l'information de volume osseux disponible pour anticiper la pose d'implants dentaires

5) pour planifier une chirurgie maxillo-faciale par déplacement des structures osseuses d'après les plans et axe de références prédéterminés.

Trois procédés sont possibles :
Le premier est, à partir d'un examen tomodensitométrique aux rayons X, de faire une reconstruction 3D du volume osseux avec la possibilité, si besoin, d'individualiser les racines. Le fichier DICOM obtenu après l'examen est ensuite traité informatiquement, grâce à un algorithme dérivé de l'algorithme des marching cubes. Le but est d'extraire après seuillage le volume d'intérêt correspondant. Ici les volumes à reconstruire ont une densité correspondant au tissu dentaire ou au tissu osseux.

Un soin tout particulier est aussi apporté à l'articulation temporo-mandibulaire pour séparer l'os de la mandibule de l'os temporal.

Dans le logiciel, son choix fait, ces paramètres déterminés, il est possible d'obtenir automatiquement un rendu de surface 3D des différentes structures. Un recalage est nécessaire et peut se faire à partir des dents qui sont à la fois visibles sur l'examen tomodensitométrique mais aussi sur les modèles 3D des arcades dentaires issues de scannage optique.

Cette technique est à privilégier dans les applications 3) et 4) mais est applicable pour toutes les situations.

Un deuxième procédé repose sur la connexion d'un échographe portable à l'ordinateur afin de réaliser une échographie 3D des os des maxillaires (mandibule et maxillaire). L'échographie se base sur l'émission et la réflexion d'ondes ultrasonores ainsi que sur l'impédance acoustique d'un milieu c'est-à-dire la capacité d'un tissu vivant à propager ou non une onde ultrasonore (résistance à la propagation). Donc, c'est en modulant l'intensité de ces ondes ultrasonores que l'on aura une information sur la présence en un endroit donné d'un tissu donné. L'impédance acoustique étant connue pour chaque tissu du corps humain il est possible de cibler l'os et d'obtenir, grâce à l'émission d'ultrasons d'une puissance adaptée et dans plusieurs directions le volume de l'os et d'entreprendre sa reconstruction informatique 3D.

Les modèles 3D des arcades dentaires issus du scannage optique sont ensuite superposés par recalage sur le modèle 3D obtenu par ultrasons en s'appuyant sur la forme des dents visibles sur les deux examens.

Les deux os maxillaire et mandibulaire sont individualisés et rattachés à leurs arcades correspondantes ce qui permet ensuite de leur appliquer les mouvements issus de la motion capture.

Le troisième procédé est le procédé inverse de la dermoplastie ou reconstruction faciale. On rappelle que le procédé de dermoplastie est employé par les anthropologues ou la police scientifique pour établir le portrait d'une personne défunte. Il consiste à apposer un volume de matière déterminé sur un crâne sec pour reconstruire le volume de la face, ces épaisseurs de matières correspondant à la couche des différents muscles masticateurs et peauciers ainsi qu'à la graisse et le derme, et qui sont relativement constantes chez les hominidés.

Le procédé mis en oeuvre dans l'invention est le procédé inverse, dit « BONE MORPHING ». Il consiste à déduire du modèle surfacique 3D du visage du patient le volume osseux sous-jacent. L'os mandibulaire est ensuite fusionné à son arcade dentaire correspondante et mis en mouvement.

Ce procédé est plus particulièrement indiqué pour les applications 1) et 2).

Applications

En permettant d'intégrer les différents déterminants de l'occlusion, le procédé tel que décrit plus haut rend possible une conduite optimale de la thérapeutique dans de nombreuses situations cliniques, notamment les bilans et traitements cliniques développés ci-dessus.

*Analyse occlusale*

L'analyse occlusale est l'étude de la répartition des dents sur les arcades dentaires, la façon dont s'engrènent les dents des deux arcades entre elles en statique et aussi en dynamique. A cet effet, il est donc nécessaire d'avoir des références propres à chaque patient. Dans ce cas les informations à obtenir sont des plans caractéristiques de la face pour en déduire le bon positionnement et la bonne orientation des dents antérieures et postérieures au sein du massif facial, pour situer le plan d'occlusion idéal grâce à une calotte occlusale déduite informatiquement. Il faut aussi quantifier et qualifier les contacts dentaires en statique et en dynamique (mastication, propulsion, latéralité) ainsi que la motilité de l'articulation temporo-mandibulaire. L'acquisition et la reproduction du mouvement de l'individu qui sont requises à cette fin sont permises par un enregistrement directement sur le patient.

*Gouttière/Orthèse interocclusale*

Une gouttière est un dispositif, généralement en matériau dur, fixé à titre temporaire sur l'arcade maxillaire ou mandibulaire et ayant pour but de modifier les rapports intermaxillaires de façon réversible. Dans ce cas l'acquisition du mouvement est importante à maîtriser. Le comparatif entre le déplacement de l'ATM et des arcades dentaires permettra de choisir numériquement la meilleure relation inter-maxillaire (positionnement de l'arcade mandibulaire par rapport à l'arcade maxillaire).

*Prothèse dentaire*

Cela consiste à restaurer ou remplacer des dents grâces à la fixation en bouche d'artifices prothétiques. L'organisation (le placement) des différentes restaurations sera guidée par rapport au plan d'occlusion sous forme de calotte occlusale déduite informatiquement. La morphologie occlusale, la partie supérieure de la dent, est conçue pour s'intégrer dans la cinématique mandibulaire du patient. L'acquisition du mouvement est importante à maîtriser. L'acquisition du modèle 3D du visage est aussi utile pour l'agencement esthétique des dents dans le secteur antérieur.

*Orthopédie dento-faciale / Orthodontie*

Il s'agit de la correction des mauvaises positions des mâchoires (ODF : orthopédie dento-faciale) ou des dents (orthodontie) afin d'optimiser l'occlusion (engrènement dentaire), ainsi que le développement des bases osseuses dans un but fonctionnel et esthétique. Cela se fait avec des appareils fixes ou amovibles. Avant la conception numérique de l'appareil d'orthodontie le résultat peut être planifié grâce à une simulation des déplacements dentaires au sein des bases osseuses. Le positionnement et l'orientation des arcades par rapport au massif facial sont recherchés, la qualité de l'engrènement dentaire en statique et en dynamique avant pendant et après le traitement enregistrés. La reconstruction 3D de l'os des maxillaires et du volume des racines à partir de l'examen tomodensitométrique à rayons X est aussi utile pour anticiper le positionnement en fin de traitement des racines entre elles ainsi que par rapport à l'os environnant disponible. Le projet esthétique du traitement orthodontique est implémenté par l'obtention du visage numérique 3D du patient.

*Chirurgie maxillo-faciale*

Dans des cas de dysmorphoses sévères, il est parfois nécessaire de recourir conjointement à une intervention de chirurgie maxillo-faciale pour compléter le traitement orthodontique. Les déplacements maxillaire et ou mandibulaire s'effectuent alors suivant une relation mandibulo-cranienne enregistrée et conservée et selon un plan de référence (le plan axio-orbitaire voisin du plan de francfort).

Enfin, il va de soi que les exemples que l'on vient de donner ne sont que des illustrations particulières en aucun cas limitatives quant aux domaines d'application de l'invention.

**REFERENCES**

WO 2013/030511

**Revendications**

**1.** Procédé de localisation des points condyliens (3) d'un patient, comprenant :

- l'obtention d'un modèle surfacique tridimensionnel du visage du patient,
- l'identification d'éléments caractéristiques du visage du patient sur ledit modèle surfacique tridimensionnel du visage du patient,
- à partir desdits éléments caractéristiques, la détermination, sur ladite image stéréoscopique, respectivement ledit modèle surfacique tridimensionnel du visage du patient, de points, axes et plans de référence du visage du patient,
- l'obtention d'un modèle tridimensionnel (Ms) de l'arcade dentaire maxillaire et d'un modèle tridimensionnel (Mi) de l'arcade dentaire mandibulaire du patient,
- le recalage des modèles tridimensionnels des arcades dentaires par rapport aux plans de référence (A, B, C) du modèle surfacique tridimensionnel du patient,
- l'enregistrement de la cinématique mandibulaire du patient,
- l'application de la cinématique mandibulaire enregistrée aux modèles tridimensionnels des arcades dentaires recalés, pour animer lesdits modèles tridimensionnels (Ms, Mi),
- à partir de ladite animation des modèles tridimensionnels (Ms, Mi) des arcades mandibulaires, la localisation du point condylien droit, respectivement gauche, ledit point condylien étant défini comme l'intersection d'un axe de rotation (R1) d'un mouvement d'ouverture-fermeture et d'un axe de rotation (R2) d'un déplacement latéral de la mandibule vers la droite, respectivement vers la gauche.

**2.** Procédé selon la revendication 1, **caractérisé en ce que** pour enregistrer la cinématique mandibulaire du patient, le patient est équipé d'un casque frontal (2000) comprenant un premier ensemble de marqueurs (2001) détectables par une caméra (1000) et une arche (4000) fixée aux dents de la mandibule portant un second ensemble de marqueurs détectables par ladite caméra, et l'on enregistre ladite cinématique mandibulaire au moyen de ladite caméra (1000).

**3.** Procédé selon la revendication 2, **caractérisé en ce que** l'étape de recalage des modèles tridimensionnels des arcades dentaires par rapport aux plans de référence du patient comprend :

- la génération d'une pluralité de points (P1-P4) sur les arcades dentaires dans le modèle surfacique tridimensionnel du visage,
- le pointage desdits points dans la bouche du patient au moyen d'un pointeur (3000) portant des marqueurs (3001) détectables par la caméra (1000),
- le repérage desdits marqueurs par la caméra lors du pointage de chaque point et la détermination de chaque point dans le référentiel de la caméra,
- la mise en correspondance des points entre le référentiel de la caméra et le modèle surfacique tridimensionnel.

**4.** Procédé selon la revendication 1, **caractérisé en ce que** l'étape de recalage des modèles tridimensionnels des arcades dentaires avec le modèle surfacique tridimensionnel du visage comprend :

   - l'obtention d'une image stéréoscopique du visage du patient sur laquelle apparaissent au moins trois dents de l'arcade dentaire maxillaire du patient,
   - la détection desdites dents par un procédé de reconnaissance faciale,
   - la mise en correspondance desdites dents sur le modèle surfacique tridimensionnel du patient et sur le modèle de l'arcade dentaire maxillaire.

**5.** Procédé selon l'une des revendications 1 à 4, dans lequel les étapes d'identification d'éléments caractéristiques du visage du patient et la détermination de points, axes et plans de référence du visage du patient comprennent :

   - l'application et la déformation d'un modèle déformable rassemblant les contours des différents éléments caractéristiques de la face pour englober les yeux, la bouche les oreilles et le nez sur le modèle surfacique tridimensionnel du visage du patient, et
   - l'application du positionnement des points, axes et plans de référence dudit modèle déformable au modèle surfacique 3D, en utilisant l'emplacement des éléments caractéristiques du visage du patient.

**6.** Système pour la mise en oeuvre du procédé selon l'une des revendications 1 à 5, comprenant :

   - un processeur,
   - une mémoire couplée au processeur,
   - un écran d'affichage couplé au processeur pour afficher des données calculées par le processeur,

le processeur étant configuré pour, à partir d'un modèle surfacique tridimensionnel du visage du patient, de modèles tridimensionnels (Ms, Mi) de l'arcade dentaire maxillaire et de l'arcade dentaire mandibulaire du patient, et d'un enregistrement de la cinématique mandibulaire du patient :

   - recaler les modèles tridimensionnels des arcades dentaires par rapport à des plans de référence du modèle surfacique tridimensionnel du patient
   - appliquer la cinématique mandibulaire enregistrée aux modèles tridimensionnels des arcades dentaires recalés, pour animer lesdits modèles tridimensionnels (Ms, Mi),
   - à partir de ladite animation des modèles tridimensionnels (Ms, Mi) des arcades mandibulaires, localiser le point condylien droit, respectivement gauche, ledit point condylien étant défini comme l'intersection d'un axe de rotation (R1) d'un mouvement d'ouverture-fermeture et d'un axe de rotation (R2) d'un déplacement latéral de la mandibule vers la droite, respectivement vers la gauche.

**7.** Système selon la revendication 6, comprenant en outre :

   - une caméra (1000),
   - un casque frontal (2000) comprenant un premier ensemble de marqueurs (2001) détectables par la caméra (1000), et
   - une arche (4000) adaptée pour être fixée aux dents de la mandibule portant un second ensemble de marqueurs détectables par ladite caméra (1000).

# FIG. 1

# FIG. 2

FIG. 3A

FIG. 3B

FIG. 5

FIG. 4

FIG. 6A

EP 4 201 322 A1

# FIG. 6B

EP 4 201 322 A1

FIG. 7A

EP 4 201 322 A1

# FIG. 7B

# FIG. 8

FIG. 9

EP 4 201 322 A1

**FIG. 10A**

**FIG. 10B**

Europäisches
Patentamt

European
Patent Office

Office européen
des brevets

# RAPPORT DE RECHERCHE EUROPEENNE

Numéro de la demande

EP 23 15 6594

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2004/015327 A1 (SACHDEVA ROHIT [US] ET AL) 22 janvier 2004 (2004-01-22) | 1,4-6 | INV.<br>A61B5/107 |
| Y | * alinéa [0012] – alinéa [0025] *<br>* alinéa [0070] *<br>* alinéa [0078] – alinéa [0079] *<br>* alinéa [0085] – alinéa [0087]; figure 4 *<br>* alinéa [0089]; figure 6 *<br>* alinéa [0091]; figure 8 *<br>* alinéa [0092]; figure 9 *<br>* alinéa [0096] – alinéa [0097] *<br>* alinéa [0098] – alinéa [0099] *<br>* alinéa [0102]; figure 16 *<br>----- | 2,3,7 | A61C19/04<br>G06T17/00 |
| X | US 2012/015316 A1 (SACHDEVA ROHIT [US] ET AL) 19 janvier 2012 (2012-01-19)<br>* alinéa [0156] – alinéa [0158] *<br>* alinéa [0167] – alinéa [0171]; figure 1 *<br>* alinéa [0254]; figures 33, 34 *<br>----- | 1,4-6 | |
| Y | MAHMOUD SEDKY ADLY ET AL: "Recording and Measuring of Jaw Movements using a Computer Vision System",<br>INTERNATIONAL JOURNAL OF COMPUTER APPLICATIONS,<br>vol. 81, no. 18,<br>1 novembre 2013 (2013-11-01), pages 975-8887, XP055485409,<br>* le document en entier *<br>----- | 2,7 | **DOMAINES TECHNIQUES RECHERCHES (IPC)**<br><br>A61B<br>A61C<br>G06T |

-/--

Le présent rapport a été établi pour toutes les revendications

| Lieu de la recherche | Date d'achèvement de la recherche | Examinateur |
|---|---|---|
| La Haye | 22 mai 2023 | Weiss-Schaber, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................................
& : membre de la même famille, document correspondant

EPO FORM 1503 03.82 (P04C02)

page 1 de 2

Europäisches
Patentamt

European
Patent Office

Office européen
des brevets

**RAPPORT DE RECHERCHE EUROPEENNE**

Numéro de la demande

**EP 23 15 6594**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DANIEL ANT?NIO FURTADO ET AL: "A specialized motion capture system for real-time analysis of mandibular movements using infrared cameras", BIOMEDICAL ENGINEERING ONLINE, BIOMED CENTRAL LTD, LONDON, GB, vol. 12, no. 1, 22 février 2013 (2013-02-22), page 17, XP021145482, ISSN: 1475-925X, DOI: 10.1186/1475-925X-12-17 * le document en entier * ----- | 2,7 | |
| Y | US 2005/267358 A1 (TUMA GREGOR [DE] ET AL) 1 décembre 2005 (2005-12-01) * alinéa [0054] * ----- | 3 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| La Haye | 22 mai 2023 | Weiss-Schaber, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.............................................................................
& : membre de la même famille, document correspondant

EPO FORM 1503 03.82 (P04C02)

**page 2 de 2**

# EP 4 201 322 A1

## ANNEXE AU RAPPORT DE RECHERCHE EUROPEENNE
## RELATIF A LA DEMANDE DE BREVET EUROPEEN NO.

EP 23 15 6594

22-05-2023

| Document brevet cité au rapport de recherche | Date de publication | Membre(s) de la famille de brevet(s) | Date de publication |
|---|---|---|---|
| US 2004015327 A1 | 22-01-2004 | EP 1624823 A2 | 15-02-2006 |
| | | EP 2165672 A2 | 24-03-2010 |
| | | US 2004015327 A1 | 22-01-2004 |
| | | US 2007207437 A1 | 06-09-2007 |
| | | WO 2004098378 A2 | 18-11-2004 |
| US 2012015316 A1 | 19-01-2012 | US 2012015316 A1 | 19-01-2012 |
| | | US 2015359614 A1 | 17-12-2015 |
| | | US 2015374460 A1 | 31-12-2015 |
| | | US 2016317250 A1 | 03-11-2016 |
| | | US 2018153646 A1 | 07-06-2018 |
| US 2005267358 A1 | 01-12-2005 | EP 1563799 A1 | 17-08-2005 |
| | | US 2005267358 A1 | 01-12-2005 |

EPO FORM P0460

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013030511 A